# EUROPEAN PATENT APPLICATION

(11) **EP 1 366 772 A1**
(43) Date of publication of application: **03.12.2003**
(21) Application number: 02711367.9
(22) Date of filing: 07.02.2002
(51) Int. Cl.: A61K 45/00, A61K 38/17, A61K 31/711, A61P 29/00

(54) **GM-CSF AND/OR DEFENSIN PROTEIN EXPRESSION REGULATORS IN EPITHELIAL CELLS COMPRISING ETS TRANSCRIPTION FACTOR OR GENE ENCODING THE SAME**

(30) Priority: 13.02.2001 JP 2001034986
(71) Applicant: HISAMITSU PHARMACEUTICAL CO., INC., Tosu-shi Saga 841-0017 (JP); Kai, Hirohumi, Kumamoto-shi, Kumamoto 862-0949 (JP)
(72) Inventor: KAI, Hirofumi, Kumamoto-shi, Kumamoto 862-0949 (JP); HISATSUNE, A., Towson, MD 212204 (US)
(74) Representative: Cresswell, Thomas Anthony
(86) International application number: JP0201012
(87) International publication number: WO02064164

(57) **Abstract**

To solve the problem of drug tolerence and side effects in antiinflammatory agents, immunosuprressive agents and antiviral agents having been put into practical use, it is intended to provide a novel therapy for inflammation and novel antiinflammatory agents with the use of an ETS transcription factor which regulates the gene expression of an inflammartory cytokine GM-CSF and a bactericidal peptide β -defensin. Namely, expression regulators for the inflammatory cytokine GM-CSF and /or β -defensin in epithelial cells and antiinflammatory agents which comprise an ETS transcription factor having a gene transcription regulatory activity or a gene encoding the same, or a substance regulating the function of ETS transcription factor or a gene encoding the same, more specifically, a transcription regulatory protein myeloid Elf-1 like factor (MEF) or a gene encoding the same, or a substance regulating the function of the MEF protein or a gene encoding the same. More specifically, inflammatory cytokine GM-CSF production inhibitors, and /or regulators for β-defensin expression due to overexpression, and antiinflammatory agents.

SEQUENCE LISTING

<110> Hisamitsu Pharmaceutical Company

<110> Kai, Hirofumi

<120> GM-CSF AND/OR DEFENSIN PROTEIN EXPRESSION REGULATORS IN EPITHELIAL
      CELLS COMPRISING ETS TRANSCRIPTION FACTOR OR GENE ENCODING THE SAME

<130> JA339329

<150> JP2001-034986
<151> 2001-02-13

<160> 2

<210> 1
<211> 663
<212> PRT
<213> Human

<400> 1

<210> 2

<211> 4190

<212> DNA

<213> Human

<400> 2
   gaattccctt tcgccggcgc cgagttcctg gcgccgctcg cccggcccgg cttccgaggg 60
   gagaggacgg gctggcgggg ctggggaccc gcgtctcggc ccccggagcg gggaccacgg 120
   agacagaccc cggcccggcg accgagctgg gcccgtgagc cactcggcct caggtcgctc 180
   ctgtggttgg tccagcccag aatgcagcct tgagcctggc ttaggccacc acctactcca 240
   gctctctcca ccccctattt tactgcagct cagggggtag gctctaggct ccaaagtacc 300
   tgggtattgt cccttcatca agaaagcccc acagctctgg agggctctga taatcccgtt 360
   gtcagctctc tgaaaagaca gcatggctat taccctacag cccagtgacc tgatctttga 420
   gttcgcaagc aacgggatgg atgatgatat ccaccagctg gaagacccct ctgtgttccc 480
   agctgtgatc gtggagcagg taccctaccc tgatttactg catctgtact cgggactgga 540
   gttggacgac gttcacaatg gcatcataac agacgggacc ttgtgcatga cccaggatca 600
   gatcctggaa ggcagttttt tgctgacaga tgacaatgag gccacctcgc acaccatgtc 660
   aaccgcggaa gtcttactca atatggagtc tcccagcgat atcctggatg agaagcagat 720
   cttcagtacc tccgaaatgc ttccagactc ggaccctgca ccagctgtca ctctgcccaa 780
   ctacctgttt cctgcctctg agcccgatgc cctgaacagg gcgggtgaca ctagtgacca 840
   ggaggggcat tctctggagg agaaggcctc cagagaggaa agtgccaaga agactgggaa 900
   atcaaagaag agaatccgga agaccaaggg caaccgaagt acctcacctg tcactgaccc 960
   cagcatcccc attaggaaga aatcaaagga tggcaaaggc agcaccatct atctgtggga 1020
   gttcctcctg gctcttctgc aagacagaaa cacctgtccc aagtacatca agtggaccca 1080
   gcgagagaaa ggcatcttca aactggtgga ctccaaagct gtgtccaagc tgtgggggaa 1140
   gcagaaaaac aagcctgaca tgaactatga gacaatgggg cgggcactaa gatactacta 1200
   ccaaagaggc atactggcca aagtggaagg gcagaggctg gtgtaccagt ttaaggagat 1260
   gcccaaggac ctggtggtca ttgaagatga ggatgagagc agcgaagcca cagcagcccc 1320
   acctcaggcc tccacggcct ctgtggcctc tgccagtacc acccggcgaa ccagctccag 1380
   ggtctcatcc agatctgccc cccagggcaa gggcagctct tcttgggaga agccaaaaat 1440
   tcagcatgtc ggtctccagc catctgcgag tctggaattg ggaccgtcgc tagacgagga 1500
   gatccccact acctccacca tgctcgtctc tccagcagag ggccaggtca agctcaccaa 1560
   agctgtgagt gcatcttcag tgcccagcaa catccaccta ggagtggccc ccgtggggtc 1620
   gggctcggcc ctgaccctgc agacgatccc actgaccacg gtgctgacca atgggcctcc 1680
   tgccagtact actgctccca ctcagctcgt tctccagagt gttccagcgg cctctacttt 1740
   caaggacacc ttcactttgc aggcctcttt cccccigaac gccagtttcc aagacagcca 1800
   ggtggcagcc ccaggggctc cactgattct cagtggcctc ccccaacttc tggctggggc 1860
   caaccgtccg accaacccgg cgccacccac ggtcacaggg gctggaccag cagggcccag 1920
   ctctcagccc cctgggactg tcattgctgc cttcatcagg acttctggca ctacagcagc 1980
   ccctagggtc aaggaggggc cactgaggtc ctcctcctat gttcagggta tggtgacggg 2040
   ggcccccatg gaggggctgc tggttcctga agagaccctg agggagctcc tgagagatca 2100
   ggctcatctt cagccacttc caacccaggt ggtttccagg ggttcccaca atccgagcct 2160
   tctgggcaac cagactttgt ctcctcccag ccgccccact gttgggctga ccccagtggc 2220
   tgaacttgag ctctcctcag gctcagggtc cctgctgatg gctgagccta gtgtgaccac 2280
   atctgggagc cttctgacaa gatcccccac cccagcccct ttctccccat tcaaccctac 2340
   ttccctcatt aagatggagc cccatgacat ataagcaaag gggtcagggc aagtgtgacc 2400
   caccaggcaa aattgagcag cattttcata gggaccgact tcagtagcac acctgcccct 2460
   gcatttcagt gggatgtcaa tacacttgac cccaagtccc ccggccctgc ctggtgtcac 2520
   tgtggccaaa cagtgcccag cttaagcatc cctggcatca gactatggcc ttcaagagca 2580
   ctagggcata tgcttttggc agcataacgg gctgacttgg tgatggaggg aaaaagcctt 2640
   gagccaggca gaagtttgtg gccagggttt gtgcagcagc tttgtgagaa gagcccttct 2700
   acctggctct atctcactgg ctgcattccc tacacaggga atttactacc ctatatgtga 2760
   atatcccctg tatgtacttg tgtgtacttg ttggtctgta tcttagtttc tttggggagg 2820
   acagggctgt agctgtgagg tcttgtctcc aagggtgtgt gtatgtctcc gtggatcagc 2880
   cacagggata gggattttgt ttttaaggga aagcattctc taattccctt tgttcatgcc 2940
   gagattcagt tgctctgaga ctatggggta caagtttgat cctccgaatc tggagatgtt 3000
   gtagagctgg aacgagtgca gagtaggaac gctttgatgc gcatgcacat tggggaagat 3060
   gcgctcctca gggacacaaa ggccgagtgg ggtaaaacca cgaagggagg gaagggaagt 3120
   cagctctggg agcagccctc actggctgga ccaaggtact cttcctggag tttgccgtgt 3180
   tagcaaccac agtcaccttg cagtcaggct ggaatcttgg gccaccccac agtgctttgc 3240
   tgtaggattt agacggggat gaagtgccct ccagcctcag agctagccac aaagccccca 3300
   gagctgaatt cattgagtat ttgtgcctag ggcttgggct gtttgtgtga taccggcccc 3360
   ccgacagaca ataggctgtg atgacacccc agtctacttc cccgatcctg ggctccctct 3420
   tgattagtag gtgacatttt ccactgtcag gcatcactgg ggctagtccg gcagcgacct 3480
   agatggggtc cacccccatt cctgctcaag catgggcacc taccacatgg tttctgctgc 3540
   tcagcctgac tgcaactcac ctcgaaggcg gaccagcctg cctctgtgat gactgcagaa 3600
   gacctccttg ggtgtaccaa tgcccctcat ctcccacttt cacacctaac cctgactcct 3660
   tcaccaagaa gacgggagtc ggcagccagg agttcccgtg gcacctctct ctcttcgtgg 3720
   ctccctgctt ccccctcccc tctttccgag gaagggtcaa cctattctct ctcaaaacca 3780
   acccctaggc caattgcctg gatctcctcc cctctccctt ctttaaacga gcttgcctcc 3840
   ctcctgccaa gtttgagggc aaggctaaga aatgtcagcc acggaaacaa ctctaatatc 3900
   tggtgacttt gggtaatgtg aatcagtgcc tgaggacctt tgctgtgtcc ttggtacaga 3960
   accatccact tgacctaact acctcccctg gccgcgctct cgctcttctc ttctttgtta 4020
   agccaacaac tatcaccctc tcctactctt ctttctccct gccccctgga gggcactgtg 4080
   tttggttgtg caaatgtatt tactatgcgt gtttccagca gttggcatta aagtgccttt 4140
   ttctaataaa atcagtttat tatgaccaaa aaaaaaaaaa aaaggaattc 4190

## Description

### Technical Field

The present invention relates to a GM-CSF and/or β-defensin protein expression regulator in epithelial cells and an anti-inflammatory agent comprising the same, wherein said expression regulator comprises an ETS transcription factor having a gene transcription regulatory activity or a gene encoding the same; or a substance regulating the function of an ETS transcription factor or a gene encoding the same; particularly a transcription regulatory protein myeloid Elf-1 like factor (referred to as MEF or MEF protein hereinbelow) or a gene encoding the same; or a substance regulating the function of the MEF protein or a gene encoding the same; and a method for screening such a functional substance. More particularly, the invention relates to an expression regulatory agent suppressing the generation of an inflammatory cytokine GM-CSF and/or activating the generation of the β-defensin protein, an anti-inflammatory agent comprising the same and a method for screening such a functional substance.

### Background Art

Excessive generation of cytokine observed in asthma, chronic obstructive pulmonary disease (referred to as COPD hereinafter), autoimmune diseases, viral diseases or cancer closely relates to the pathogenesis and pathological conditions thereof. It has been known that the inflammatory cytokines such as granulocyte macrophage colony stimulating factor (referred to as GM-CSF hereinafter), tumor necrosis factor α (referred to as TNFα hereinafter) and interleukin-1 (referred to as IL-1 hereinafter) over-expressed due to the chronic airway inflammation typically including asthma and COPD, for example, trigger the enhanced inflammatory reactions caused by accumulation/activation of inflammatory cells, and tissue modifications i.e. airway remodeling, which enhance the severity of pathological states.

GM-CSF is an inflammatory cytokine of a molecular weight of about 14 kDa as generated in and secreted from epithelial cell, macrophage, T lymphocyte, endothelial cell and fibroblast. The target cells thereof are diverse and include, for example, a hematopoietic stem cell, erythroblast, granulocyte, and macrophage. GM-CSF plays important roles in the proliferation, differentiation and functional activation of those cells. Through the functional activation of those cells, GM-CSF further enhances the generation of other inflammatory cytokines such as TNFα and IL-1 and induces the cytokine cascade, thereby indicated a potential therapeutic applicability for various inflammatory disease by suppressing the function or the expression of GM-CSF. In order to obtain an anti-inflammatory function by suppressing the functions of GM-CSF, the investigations using the neutralizing antibodies have been carried out, however, revealed no significant effects. Many attempts have been made to develop a pharmaceutical agent having an anti-inflammatory function by suppressing the expression or activity of NF-κB which regulates the gene expression of inflammatory cytokines. However, such a pharmaceutical agent is problematic in terms of the specificity. It is therefore suggested that an anti-inflammatory therapy via the specific suppression in the expression amount of GM-CSF per se, namely the regulation of the expression of the GM-CSF gene and/or protein would be useful.

On the other hand, there lies a concern of deterioration in the biological protection mechanism due to the suppression of the expression of anti-inflammatory cytokines, which cause the suppression of immune potency against such as infections. A low molecular bactericidal peptide defensin is an amphiphatic molecule having both positively charged hydrophilic (basic) and hydrophobic domains. Allowing the hydrophilic domain to bind to a negatively charged molecule on the bacterial membrane followed by inserting the hydrophobic domain into the bacterial membrane, consequently the permeability of the bacterial membrane being activated, thereby inhibit the bacterial metabolism so that defensin can exert bactericidal function. The above described biological protection mechanism can be resumed by the expression of the defensin gene and/or protein or the enhancement of the activation thereof at an inflammatory site.

In view of above discussion, it would be significant for anti-inflammatory therapy to regulate, at gene level, the expression of a factor relating to an inflammatory reaction and a factor controlling the biological protection mechanism.

Transcription factors bind to a specific sequence of the gene expression region of a gene to interact with the primary transcription factor, thereby regulate the gene expression at the transcription level. With respect to an ETS (E26 transforming specific) transcription factor group, approximately 50 species ranging from drosophilae to humans have been reported. It has been known that the ETS transcription group has a DNA binding region comprising 85 amino acids having a high homology, commonly recognizes a base sequence to which the ETS transcription factor group binds (hereinafter referred to as ETS binding site), and regulates the expression of a gene specifically expressing in hemocytes. Further, it has been reported the ETS transcription factor group not only has a gene expression regulatory activity but also participates in differentiation, proliferation and functional expression of many cells including hemocytes.

In recent years, it is disclosed that the ETS transcription factor group is involved in the regulation of the gene expression of GM-CSF and defensin in hemocytes, however, such a mechanism in epithelial cells has not been revealed yet. If the involvement of the ETS transcription factor group in the regulation of the expression of GM-CSF gene due to the chronic inflammations typically including asthma in epithelial cells and in the regulation of the expression of the defensin gene due to the infection is revealed, by which the expression mechanism can be regulated and thereby expected a development of a novel therapeutic method for treating these disorders.

### Disclosure of the Invention

In view of the above discussion, the present invention provides a novel anti-inflammatory therapeutic method and agents, by using an ETS transcription factor which regulates the expression of the genes of an inflammatory cytokine GM-CSF and a bactericidal peptide β-defensin in order to solve the problems of drug resistance and side effects of anti-inflammatory agents, immune suppressors and anti-viral agents which have been in practical use.

### Brief Description of the Drawing

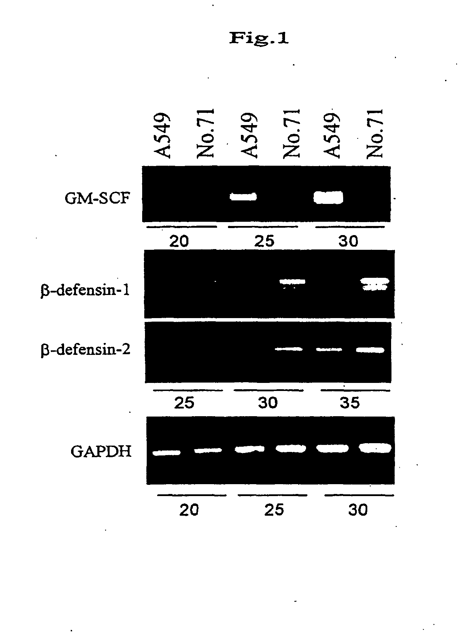

Fig. 1 is a photopicture in lieu of a drawing, depicting the RT-PCR results of the expression of GM-CSF and β-defensin genes in a human pulmonary epithelium-derived cell line (A549) and MEF gene stable overexpression cell strain (No.71 strain) as prepared from the cell line A549.

### Best Mode for Carrying out the Invention

The inventors have made investigations on the ETS transcription factor, and consequently found that an ETS transcription factor MEF specifically expressed in submucosal gland in airway epithelium can specifically suppress the expression of the GM-CSF gene and enhance the expression of the β-defensin gene.

Namely, the invention provides the following 1 to 13.
1. The expression regulator of an inflammatory cytokine GM-CSF and/or the defensin protein in epithelial cells, comprising an ETS transcription factor or a gene encoding the same, or a substance regulating the function of an ETS transcription factor or a gene encoding the same.
2. The expression regulator as described in No.1, wherein the ETS transcription factor or a gene encoding the same is the MEF protein or a gene encoding the same.
3. The expression regulator as described in No.1 or 2, wherein the substance regulating the function of an ETS transcription factor or a gene encoding the same is a substance regulating the function of the MEF protein or a gene encoding the same.
4. The expression regulator as described in No.2 or 3, wherein the MEF protein is a protein comprising an amino acid sequence of SEQ ID NO:1, or an amino acid sequence wherein one or more amino acids are substituted, deleted or added.
5. The expression regulator as described in No.2 or 3, wherein the gene encoding the MEF protein is a nucleotide comprising a nucleotide sequence of SEQ ID NO:2, or a nucleotide sequence wherein one or more amino acids are substituted, deleted or added, or a nucleotide comprising a nucleotide sequence which can hybridize to said nucleotide sequences under stringent conditions.
6. The expression regulator as described in any one of Nos.1 to 5, wherein the regulatory activities in epithelial cells comprise suppressing the generation of the inflammatory cytokine GM-CSF and/or activating the generation of the β-defensin protein.
7. The expression regulator as described in any one of Nos.1 to 6, wherein the expression regulator is an anti-inflammatory agent.
8. A method for screening a substance capable of regulating the expression of GM-CSF and/or the defensin protein in cells, wherein used a cell transfected therein a gene encoding an ETS transcription factor, and a test substance is added to around the cell to measure the amount of GM-CSF and/or the defensin protein expressed in the cell.
9. The method as described in No.8, wherein the gene encoding an ETS transcription factor is a gene encoding the MEF protein.
10. The method as described in No.8 or 9, comprising a method for screening a substance having an anti-inflammatory function.
11. An anti-inflammatory agent comprising a substance regulating the function of an ETS transcription factor or a gene encoding the same.
12. The anti-inflammatory agent as described in No.11, wherein the substance regulating the function of an ETS transcription factor or a gene encoding the same is a substance regulating the MEF protein or a gene encoding the same.
13. The anti-inflammatory agent as described in No.11 or 12, wherein the substance regulating the function of an ETS transcription factor or a gene encoding the same is a substance obtained by a method described in any one of Nos.8 to 10.

The invention relates to an expression regulator of an inflammatory cytokine GM-CSF and/or the defensin protein in epithelial cells, comprising an ETS transcription factor or a gene encoding the same, or a substance regulating the function of an ETS transcription factor or a gene encoding the same; and an anti-inflammatory agent comprising the same. That is, the invention relates to a pharmaceutical composition which regulates the expression of GM-CSF and/or the defensin protein in epithelial cells, comprising an ETS transcription factor or a gene encoding the same, or a substance regulating the function of an ETS transcription factor or a gene encoding the same, more specifically a pharmaceutical composition useful as an anti-inflammatory agent for regulating the expression of GM-CSF and/or the defensin protein in epithelial cells, a method for curing, preventing or treating an inflammation involved in the expression of GM-CSF and/or the defensin protein in epithelial cells, comprising administering an effective amount of the pharmaceutical composition to patients suffering from inflammatory diseases; and the use of an ETS transcription factor or a gene encoding the same or a substance regulating the function of an ETS transcription factor or a gene encoding the same for producing the pharmaceutical composition.

Further, the invention relates to a method for screening a substance capable of regulating the expression of GM-CSF and/or the defensin protein in epithelial cells, comprising using the cell transfected therein a gene encoding an ETS transcription factor and adding a test substance to or around the cell in order to measure the amount of GM-CSF and/or the defensin protein expressed in the cell; and relates to a substance screened by said method, which is capable of regulating the expression of GM-CSF and/or the defensin protein in epithelial cells.

The invention is now described in detail hereinbelow.

### (MEF protein and MEF gene)

MEF is a transcription factor of a molecular weight of about 100 kDa and with 663 amino acids, as isolated from the mRNA of CMK as a human megakaryocytic cell line by Miyazaki, et al. in 1996, which belongs to the ETS transcription factor group. It is also reported that MEF regulates the expression of GM-CSF and interleukin-3 in hemocytes (Miyazaki, Y., et al., Oncogene, 13, 1721-1729, 1996). Additionally, the present inventors already showed that MEF regulated the expression of lysozyme with an antibacterial activity in epithelial cells (Kai, H., et al., J. Biol. Chem., 274(29), 20098-20102, 1999)). In other words, the inventors reported that an ETS transcription factor PU.1 regulated the expression of the lysozyme gene in hemocytes and also demonstrated that the expression thereof in epithelial cells was regulated by an ETS transcription factor MEF.

The inventors prepared a cell line highly expressing the MEF gene constantly therein by inserting the MEF gene into the epithelial cells so as to carry out detailed examination of the influence of MEF i.e. one of ETS transcription factors on epithelial cells on the basis of the assumption that the expression of gene in hemocytes regulated by PU.1, would be regulated by MEF in epithelial cells.

The expression of GM-CSF gene and the β-defensin gene in a human pulmonary epithelium-derived cell line (A549) and MEF gene stable overexpression cell strain (No.71 strain) prepared by using the cell line A549 was examined by RT-PCR. The results are shown in Fig. 1, which is a photopicture in lieu of a drawing. In Fig. 1, the abscissa represents the number of cycles in RT-PCR. In other words, the result after 20 cycles is indicated at the position of numerical figure 20; 25, after 25 cycles; 30, 35, indicated in the same manner. The result in A549 or the parent strain is shown on the left side of each pair, while the result in MEF gene stable overexpression cell strain (No.71 strain) is shown on the right. The top column in Fig.1 shows the results of GM-CSF; the two columns in the middle show the results of β-defensin, wherein the upper column shows the results of β-defensin-1 and the lower column shows the results of β-defensin-2. The bottom column in Fig. 1 shows the result of the control GAPDH for checking the color development.

Fig.1 shows remarkable expression of GM-SCF in the parent strain A549, while the expression of GM-SCF is markedly suppressed in the No.71 strain. On the other hand, no or very little expression of β-defensin is observed in the parent strain A549, while the remarkable expression is observed in the No.71 cell strain. Particularly, with regard to the expression of β-defensin-1, the difference between the two strains is significant. The base sequences of the PCR products obtained through this experiment were identified by the cycle sequence method.

Consequently, it was found that the expression of the GM-CSF gene was remarkably reduced in MEF gene stable overexpression cell strain (No.71 strain) in comparison to the parent strain, while the expression of β-defensin-1 and -2 was remarkably enhanced.

This apparently indicates that the introduction of an ETS transcription factor, specifically MEF can regulate the expression of GM-CSF, and β-defensin-1 and -2 in epithelial cells.

Thus, the ETS transcription factor of the invention, specifically MEF is useful for the treatment of diseased conditions wherein the continuous proliferation and progression are mediated by GM-CSF i.e. encompassed in the stages of the biochemical pathways, or requiring GM-CSF. It is also useful for the treatment of diseases requiring the expression of β-defensin-1 or -2. Such conditions include diseases involving excess generation of macrophage and granulocyte, for example, temporal arthritis, polyarthritis nodosa, systemic lupus erythematosus, nephritis in various forms, atheroma arteriosclerosis, Kaposi sarcoma of AIDS, mesangium proliferative nephritis, eosinophilic pneumonia, psoriasis, and chronic arthritis.

The ETS transcription factor of the invention includes various transcription factors capable of recognizing the ETS-binding site. Among them, MEF is preferable. MEF has the amino acid sequence of SEQ ID NO:1. MEF usable in the invention is not limited to MEF having such an amino acid sequence but includes any MEF having an activity of regulating the expression of at least one of GM-CSF, β-defensin-1 and β-defensin-2. Preferably, any MEF of an amino acid sequence capable of recognizing the ETS-binding site is satisfactory. Thus, the MEF of the invention includes a polypeptide having an amino acid sequence of SEQ ID NO:1 wherein one or more amino acids are substituted, deleted or added. Such polypeptides, being mutants of MEF proteins, are encompassed within the scope of the invention as long as they have the above activity.

Similarly, the gene encoding the ETS transcription factor of the invention includes genes encoding the aforementioned ETS transcription factor of the invention. The preferable gene encoding the ETS transcription factor of the invention includes the gene encoding above described MEF. An example of the gene encoding MEF is shown as the gene of SEQ ID NO:2, however, it is not limited to the gene having said base sequence. For example, the gene of the invention also includes a polynucleotide having an amino acid sequence of SEQ ID NO:2 wherein one or more amino acids are substituted, deleted or added.

Further, the gene of the invention also includes a gene having a base sequence which can hybridize to a gene having said base sequence under stringent conditions.

A sugar chain is added to many of ordinary proteins, and such an addition can be regulated by converting one or more amino acids. A polypeptide regulated with a sugar chain addition in the amino acid sequence of SEQ ID NO:1 is also encompassed within the invention, as long as it has the above activity. Further, a polynucleotide encoding the above described polypeptide is also encompassed within the invention.

Further, the invention includes an anti-inflammatory therapeutic method or agent by using the substances such as proteins, peptides, organic compounds and steroids, which can enhance the expression of an ETS transcription factor in epithelial cells or a gene encoding the same, preferably the MEF protein or the gene thereof.

### (Gene therapy)

The invention can be used in gene therapy against the various diseases listed above by incorporating MEF into a therapeutic vector.

In the invention, the vector used in the gene therapy includes, but not limited to, the vectors derived from recombinant vaccine virus, polio virus, influenza virus, adenovirus, adeno-associated virus, herpes virus, HIV virus, Sendai virus and the like. Further, sequences relating to the gene expression, such as appropriate promoters, replication origins, selective markers, RNA splicing sites, and polyadenylated signals are introduced into said vectors.

The invention is used as gene therapeutic agents in a usual manner by incorporation into the vectors. That is, in case of performing gene therapy, it is advisable that the recombinant virus vector is contacted with target cells in therapy or inserted into an expression vector such as plasmid vector to transfect the same into target cells. The transfection can then be performed by known methods such as a calcium phosphate method, a liposome method, an electroporation method and DEAE-dextran method.

The term "oligonucleotide" used in the specification means an oligonucleotide formed from a naturally occurring base and a sugar moiety bound by an inherent phosphodiester bond and its analogues. Accordingly, a first group encompassed within the term includes naturally occurring species, or synthetic species generated from naturally occurring subunits or homologues thereof. It refers to a base-sugar combination bound to subunits through a phosphodiester bond or other bond. A second group of the oligonucleotide is analogues thereof, which function similarly to the oligonucleotide but have residues with moiety never occurring naturally. These include oligonucleotides with phosphate groups for enhancing stability, sugar moieties, and chemical modifications at 3' and 5' terminals. Examples thereof include oligophosphorothioate where one of oxygen atoms in the phosphodiester group between nucleotides is substituted with sulfur, or oligomethylphosphonate where it is substituted with -CH₃. The phosphodiester bond may be substituted with other nonionic and achiral structures. As for oligonucleotide analogues, species including modified bases, namely, purines and pyrimidines other than those usually found in nature, may be used. Such oligonucleotides are also included in the invention as the DNA derivatives so long as they exhibit the same function as the antisense DNA of the invention.

In the invention, the target portion of mRNA to which the oligonucleotide is hybridized is preferably a transcription initiation site, a translation initiation site, an intron-exon binding site or a 5'-cap site. In consideration of a secondary structure of mRNA, a site free from steric hindrance has to be selected.

### (Production and use of MEF)

MEF of the invention can be produced by transforming host cells such as procaryotic cells or eucaryotic cells with DNA described in SEQ ID No. 2 and an expression vector having transfected therein an appropriate sequence involved in gene expression such as promoter, replication origin, selective marker, RNA splicing site and polyadenylation signal to express MEF gene in the host cells. Further, the MEF of the invention can be produced by ligating a gene encoding a different protein to the DNA relating to the invention to allow the expression of a fusion protein to expedite purification of MEF, increase the amount expression, or to carry out an appropriate treatment at the purification step to excise the generated MEF.

Further, mutant MEF can also be produced by mutation of one or more nucleotides of DNA described in SEQ ID No.2, addition of another nucleotide thereto, excision of a part of 3' or 5'-side or deletion of one or more nucleotides therein.

Among the hosts for use in the expression system, the prokaryotic host cell includes for example Escherichia coli and Bacillus subtilis. Among eucaryotic organisms, further, the eucaryotic host cell includes, for example, yeast and Myxomycetes. Instead, insect cells such as Sf9 may also be used as the host cell. Additionally, the host cell derived from animal cells includes, for example, COS cell and CHO cell.

In the invention, MEF produced by the above method can be used after separation from the inside or outside of the host cells and purification. For separation and purification of MEF, the common methods for separating and purifying the proteins can be used. The methods such as various kinds of chromatographies, ultrafiltration, salting, dialysis can be selected and used in combination upon requirement.

In the invention, ETS transcription factor of the invention, preferably MEF can be administered by intravenous administration, local administration to the affected part, oral administration or the like. In the administration, MEF is formulated into preparations appropriate for the administration by adding thereto pharmaceutically acceptable additives such as carriers, excipients, stabilizers and solubilizers.

In accordance with the invention, furthermore, an antibody recognizing an oligopeptide having at least five sequential amino acids in the amino acid sequence (SEQ ID No: 1) of MEF can be prepared. Specifically, the antibody can be obtained by immunizing an animal with an oligopeptide as an antigen, collecting the antibody generated in vivo and then purifying the antibody. The antibody includes polyclonal antibody and monoclonal antibody, and methods for purifying these antibodies are known to those skilled in the art. Any anti-MEF antibodies obtained in such a manner can be used for detection and quantitative determination of MEF in the various immunological assays such as enzyme immunoassay e.g. ELISA, radio-immunoassay, and fluorescence immunoassay, or for MEF purification on columns.

As for the active ingredient of the invention, not only the ETS transcription factor or a gene encoding the same but also a substance regulating the ETS transcription factor or a gene encoding the same can be used because such a substance can produce the same results in vivo or in vitro as described above.

The substance regulating the ETS transcription factor or a gene encoding the same in accordance with the invention may be any substance capable of regulating the ETS transcription factor or a gene encoding the same in vivo or in vitro and includes substances such as proteins, peptides, organic compounds and steroids, which can regulate the expression. The term "regulating the function" according to the method of the invention means suppressing or enhancing the function or, in a certain condition, means both. However, it is preferable that the substance has a function either of the two.

The invention provides a method for screening these substances. Specifically, the invention provides a method for screening a substance capable of regulating the expression of GM-CSF and/or the defensin protein in cells, wherein a cell transfected therein a gene encoding an ETS transcription factor is used, and a test substance is added to or around the cell to measure the amount of GM-CSF and/or the defensin protein expressed in the cell, as well as the substance screened by the method, which can regulate the expression of GM-CSF and/or the defensin protein in cells.

As the cell having transfected therein the gene encoding the ETS transcription factor in the invention, a cell having a gene encoding a naturally occurring ETS transcription factor may be used as it is. Preferably, a transformant obtained by transfecting a gene encoding an ETS transcription factor into an appropriate expression vector and subsequently introducing the resulting vector into an epithelial cell or various microbial cells can be used. The microbial cells which can be used in this method are procaryotic host cells or host cells of eucaryotic microbes.

According to the method of the invention, it is possible to identify the substance whether it has the same objet or not, by adding a test substance of various concentrations to or around a cell transfected therein a gene encoding the ETS transcription factor, and measuring the amount of the ETS transcription factor, or GM-CSF and/or the defensin protein expressed therein.

The substance obtained by this method of the invention is a substance capable of regulating the amount of GM-CSF and/or the defensin protein expressed in cells, preferably in epithelial cells and can suppress or enhance the function derived from GM-CSF and/or the defensin protein. More specifically, the substance is useful as a therapeutic agent, preventing agent or treating agent of the various inflammatory diseases described above. Accordingly, the invention includes substances capable of regulating the amount of GM-CSF and/or the defensin protein expressed, which are obtained by the method.

Further, the invention includes pharmaceutical compositions such as therapeutic agents, preventive agents or treating agents containing the substance screened by the method as the active ingredient, a therapeutic method for treating inflammation using the same and the use for production of the pharmaceutical compositions.

### Examples

The invention is illustrated more specifically below by referring to Examples. However, the invention is not limited by these Examples. Origins of reagents are described for convenience sake, and do not limit the invention.

### Example 1 (Preparation of MEF gene stable overexpression cell strain)

### 1. Incubation of human pulmonary epithelium-derived A549 cell strain

Human pulmonary epithelial cell strain A549 was statically incubated in a cell proliferation culture solution obtained by adding 10% FBS (fetal bovine serum)(Hyclone, Lot No. AGB 6235) and antibiotics (penicillin G (100 units/ml) and streptomycin (100 µg/ml) to a basic culture solution (Dulbecco's Modified Eagle Medium, pH=7.4) under conditions of 5% CO₂ at 37°C.

### 2. Preparation of gene

100 µg of an expression vector (pCB6) having MEF cDNA incorporated therein was digested with restriction enzyme ApaL I to prepare linear DNA.

### 3. Transfection of MEF gene into cells

Cells in a subconfluent state (from 50 to 60%) were recovered by trypsin digestion, and suspended in a site mix solution (120 mM KCl, 0.15 mM CaCl₂, 10 mM K₂HPO₄/KH₂PO₄, pH 7.6, 25 mM Hepes, pH 7.6, 2 mM EGTA, pH 7.6, 5 mM MgCl₂, 2 mM ATP, pH 7.6; 5 mM glutathione; pH adjusted with KOH) containing the foregoing DNA (100 µg). The suspension was then poured into cuvettes (ELECTROPORATION CUVETTES PLUSTM, 2 mm gap, BTX), and allowed to stand on ice for 10 minutes. Subsequently, electric shock (500 V, 1,350 mF) was applied with a pulser (ELECTRO CELL MANIPURATION ECM 600, BTX), and the resulting suspension was again allowed to stand on ice for 10 minutes, and incubated under the condition of 5% CO₂ for 37°C. Incidentally, the amount of DNA used was measured from an absorbance of UV (260 nm), and DNA having a purity of 85% or more was used.

### 4. Determination of MEF gene stable overexpression cell strain

When the cells became nearly 50%-confluent, G418 (Nacalai Tesque) was added to a final concentration of 1.0 mg/ml, and the mixture was further incubated for one week. Incidentally, G418 was used at a concentration at which untransfected cells were destroyed in one week. One week later, colonies of survival cells were recovered, and incubated in separate 60-mm culture dishes, and the selection with G418 was conducted again. Finally, the expression of MEF gene in each cell was identified by northern blotting analysis and RT-PCR to obtain MEF gene stable overexpressing cell strain (No. 71 strain).

### Example 2 (Expression of GM-CSF and β-defensin in the cell line)

The expression of GM-CSF and β-defensin in MEF gene stable overexpression cell strain was identified by RT-PCR using the TaKaRa RT-PCR kit (TaKaRa RNA PCR kit (AMV) Ver.2.1) in accordance with the protocol thereof. A reverse transcription (at 45°C for 45 minutes, at 99 °C for 5 minutes and at 5 °C for 5 minutes) was performed using 1 µg of the all RNAs extracted from the cell. Subsequently, PCR (at 94 °C for one minute, at 50 °C for one minute, at 72 °C for 1.5 minutes - 40 cycles; at 72 °C for 20 minutes - one cycle) was performed adding the primers for GM-CSF and β-defensin to the reaction solution. Herein, the base sequence of the resulting PCR product was identified by the cycle sequence method. The results are shown in Fig. 1.

The results of these experiments show the significant suppression in expression of the GM-CSF gene in the cell line compared to the parent cell line, while show the enhancement in expression of β-defensin gene though the expression levels differ depending on its subtypes.

### Industrial Applicability

GM-CSF is one of the factors causing a severity of diseased conditions due to excess inflammation, as well as a substance inducing inflammatory reaction. The invention reduces the severity of inflammatory condition by suppressing the expression of GM-CSF. Additionally, the enhancement of the expression of an antimicrobial peptide i.e. β-defensin may reduce the infectious condition caused by the suppression of inflammatory reaction, thereby maintained the biological homeostasis.

## Claims

1. A GM-CSF and/or defensin protein expression regulator in epithelial cells, comprising an ETS transcription factor or a gene encoding the same, or a substance regulating the function of an ETS transcription factor or a gene encoding the same.

2. The expression regulator according to claim 1, wherein the ETS transcription factor or the gene encoding the same is a MEF protein or a gene encoding the same.

3. The expression regulator according to claim 1 or 2, wherein the substance regulating the function of the ETS transcription factor or the gene encoding the same is a substance regulating the function of the MEF protein or the gene encoding the same.

4. The expression regulator according to claim 2 or 3, wherein the MEF protein is a protein comprising an amino acid sequence of SEQ ID NO:1, or an amino acid sequence wherein one or more amino acids are substituted, deleted or added.

5. The expression regulator according to claim 2 or 3, wherein the gene encoding the MEF protein is a nucleotide comprising a nucleotide sequence of SEQ ID NO:2, or a nucleotide sequence wherein one or more nucleotides are substituted, deleted or added, or a nucleotide comprising a nucleotide sequence which can hybridize to said nucleotide sequences under stringent conditions.

6. The expression regulator according to any one of claims 1 to 5, wherein the regulatory activities in epithelial cells comprise suppressing the generation of the inflammatory cytokine GM-CSF and/or activating the generation of the β-defensin protein.

7. The expression regulator according to any one of claims 1 to 6, wherein the expression regulator is an anti-inflammatory agent

8. A method for screening a substance capable of regulating the expression of GM-CSF and/or the defensin protein in cells, wherein used a cell transfected therein a gene encoding an ETS transcription factor, and a test substance is added to or around the cell to measure the amount of GM-CSF and/or the defensin protein expressed in the cell.

9. The method according to claim 8, wherein the gene encoding an ETS transcription factor is a gene encoding the MEF protein.

10. The method according to claim 8 or 9, comprising a method for screening a substance having an anti-inflammatory function.

11. An anti-inflammatory agent comprising a substance regulating the function of an ETS transcription factor or a gene encoding the same.

12. The anti-inflammatory agent according to claim 11, wherein the substance regulating the function of an ETS transcription factor or a gene encoding the same is a substance regulating the MEF protein or a gene encoding the same.

13. The anti-inflammatory agent according to claim 11 or 12, wherein the substance regulating the function of an ETS transcription factor or a gene encoding the same is a substance obtained by the method according to any one of claims 8 to 10.
